(19) Europäisches Patentamt
European Patent Office
Office européen des brevets

(11) EP 1 070 724 B1

(12) **EUROPÄISCHE PATENTSCHRIFT**

(45) Veröffentlichungstag und Bekanntmachung des Hinweises auf die Patenterteilung:
**05.03.2003 Patentblatt 2003/10**

(51) Int Cl.[7]: **C07F 9/59**, C07F 9/572, C07B 39/00

(21) Anmeldenummer: **00114363.5**

(22) Anmeldetag: **05.07.2000**

(54) **Tetrakis(pyrrolidino/piperidino)phosphoniumsalze enthaltende Mischungen**

Mixtures comprising tetrakis(pyrrolidino/piperidino)phosphonium salts

Mélanges contenant des sels de tétrakis(pyrrolidino/piperidino)phosphonium

(84) Benannte Vertragsstaaten:
**AT BE CH CY DE DK ES FI FR GB GR IE IT LI LU MC NL PT SE**

(30) Priorität: **23.07.1999 DE 19934594**

(43) Veröffentlichungstag der Anmeldung:
**24.01.2001 Patentblatt 2001/04**

(73) Patentinhaber: **Clariant GmbH**
**65929 Frankfurt am Main (DE)**

(72) Erfinder:
- **Schiemenz, Berthold, Dr.**
**65929 Frankfurt (DE)**
- **Wessel, Thomas, Dr.**
**61138 Niederdorfelden (DE)**
- **Pfirmann, Ralf, Dr.**
**64347 Griesheim (DE)**
- **Beck, Andreas**
**63825 Westerngrund (DE)**
- **Hahn, Walter**
**65934 Frankfurt (DE)**

(56) Entgegenhaltungen:
**WO-A-97/32832** **WO-A-98/22413**
**WO-A-98/32532** **WO-A-99/26950**

- **KOIDAN G N ET AL: "SOME PROPERTIES OF PHOSPHORIMIDIC TRIAMIDES" JOURNAL OF GENERAL CHEMISTRY USSR, Bd. 52, Nr. 9, 20. Februar 1983 (1983-02-20), Seiten 1779-1787, XP002065181**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die Erfindung betrifft Aminophosphoniumsalze enthaltende Mischungen (Stoffgemische), ihre Herstellung und Verwendung.

Aminophosphoniumverbindungen finden, wie aus WO 98/32532 und WO 98/22413 hervorgeht, bei Herstellung von Fluor enthaltenden Verbindungen mittels einer Halogen-Fluor-Austauschreaktion (Halex-Reaktion) als Katalysatoren Anwendung. Das in WO 98/32532 und WO 98/22413 verwendete Tetrakis-(diethylamino)-phosphoniumbromid liefert zwar gute Ergebnisse, weist aber eine sehr hohe dermale Toxizität auf. Die sehr hohe dermale Toxizität von < 50 mg/kg Körpergewicht steht einer technischen Anwendung allerdings entgegen.

Es besteht die Aufgabe, neue, in hohen Anteilen Tetrakisaminophosphoniumsalze enthaltende Mischungen bereitzustellen, die sich als Katalysator oder Bestandteil von Katalysatorsystemen für Phasentransferreaktionen, insbesondere für Halogen-Fluor-Austauschreaktionen eignen, eine geringere dermale Toxizität besitzen und die die unter Verwendung von Tetrakis(diethylamino)-phosphoniumbromid erzielbaren Ergebnisse erreichen oder sogar noch übertreffen. Diese Mischungen sollen sich zudem auf vergleichsweise einfache Weise mit einem vertretbaren Aufwand auch in technischen Mengen herstellen lassen.

Diese Aufgabe wird überraschenderweise gelöst durch Mischungen enthaltend 70 bis 99,5 Gew.-% einer Verbindung der Formel $(R)_4P^+X^-$ (1) und 30 bis 0,5 Gew.-% einer Verbindung der Formel $(R)_3P{=}O$ (2) wobei R jeweils für einen Rest

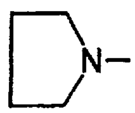

oder

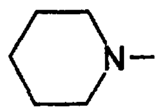

und $X^-$ für ein anorganisches oder organisches Anion oder das Äquivalent eines mehrwertigen anorganischen oder organischen Anions steht.

Die Mischungen enthalten die Verbindungen der Formel $(R)_4P^+X^-$ (1), nämlich die entsprechenden Tetrakis (pyrrolidino)phosphoniumsalze bzw. die Tetrakis(piperidino)phosphoniumsalze in hohen Anteilen. Die in den Mischungen enthaltenen Verbindungen $(R)_3P{=}O$ wirken sich überraschenderweise auf die katalytische Wirksamkeit nicht negativ aus, so daß die Mischungen unmittelbar als Katalysator oder Katalysatorkomponente eingesetzt werden können.

Die dermale Toxizität liegt sowohl für Tetrakis(pyrrolidino)phosphoniumchlorid als auch für Tetrakis(piperidino) phosphoniumchlorid in unerwarteter Weise signifikant tiefer als die von Tetrakis(diethylamino)phosphoniumbromid, obwohl keine gravierenden Unterschiede hinsichtlich Molekülstruktur/Molekülgröße und Molekülmasse des Tetrakisphosphonium-Kations vorliegen. Die dermale Toxizität von Tetrakis(piperidino)phosphoniumchlorid beträgt ~ 200 mg/kg Körpergewicht, die von Tetrakis(pyrrolidino)phosphoniumchlorid beträgt ~ 390 mg/kg Körpergewicht und ist somit erheblich niedriger als die von Tetrakis(diethylamino)-phosphoniumbromid. Die Angaben für die dermalen Toxizitäten von Tetrakis(diethylamino)phosphoniumbromid, Tetrakis(piperidino)phosphoniumchlorid und Tetrakis(pyrrolidino) phosphoniumchlorid gehen auf eigene Messungen zurück.

Die vorliegende Erfindung betrifft insbesondere Mischungen enthaltend 75 bis 99, bevorzugt 80 bis 98, besonders bevorzugt 85 bis 95 Gew.-% der Verbindung $(R)_4P^+X^-$ (1) und 25 bis 1, bevorzugt 20 bis 2, insbesondere bevorzugt 15 bis 5 Gew.-% der Verbindung $(R)_3P{=}O$ (2). R steht - wie zuvor bereits ausgeführt - sowohl in den Verbindungen (1) als auch in den Verbindungen (2) für einen Pyrrolidino- oder Piperidinorest, insbesondere für einen Pyrrolidinorest.

In den Verbindungen der Formel (1) steht $X^-$ für $F^-$, $Cl^-$, $Br^-$, $J^-$, $ClO_4^-$, $BF_4^-$, $PF_6^-$, $NO_3^-$, $HSO_4^-$, ½ $SO_4^{2-}$, $H_2PO_4^-$, ½ $HPO_4^{2-}$, $^1/_3$ $PO_4^{3-}$, R"-COO$^-$, wobei R" einen Alkylrest mit 1 bis 9 C-Atomen, einen Phenylrest, Benzylrest oder Naphthylrest bedeutet, R"'-$SO_3^-$, wobei R"' einen Alkylrest mit 1 bis 18 C-Atomen, einen Phenylrest, Tolylrest oder Naphthylrest bedeutet, $HCO_3^-$, ½ $CO_3^{2-}$ oder für ½ $C_6H_4(COO^-)_2$.

$X^-$ bedeutet insbesondere $F^-$, $Cl^-$, $Br^-$, $J^-$, $BF_4^-$, $PF_6^-$ oder ½ $SO_4^{2-}$, bevorzugt $F^-$, $Cl^-$ oder $Br^-$, insbesondere bevorzugt $Cl^-$.

Die Mischungen fallen üblicherweise als Stoffgemische an, die zu 95 bis 100, insbesondere 96 bis 99,5, vorzugsweise 97 bis 99 Gew.-% aus der Mischung enthaltend die Verbindungen $(R)_4P^+X^-$ und $(R)_3P{=}O$ und zu 5 bis 0, insbesondere 5 bis 0,5, vorzugsweise 3 bis 1 Gew.-% flüchtigen Bestandteilen bestehen. Als flüchtige Bestandteile können beispielsweise Reste nicht umgesetzter Einsatzstoffe und Lösungsmittel noch vorhanden sein. Es ist allerdings möglich, diese flüchtigen Bestandteile weitestgehend oder vollständig abzutrennen.

G.N. Koidan et al., beschrieben in J. Gen. Chem. USSR (Engl. Transl.) 52, 1982, Seite 1779 bis 1787 eine

mehrstufige Herstellung von Tetrakis(piperidino)-phosphoniumbromid. Hierbei wird zunächst eine Verbindung der Formel $(R_2N)_3P^+HalHal^-$ mit Ammoniak umgesetzt und aus dem sich bildenden Reaktionsprodukt unter Wasserabspaltung ein Triamid einer Iminophosphorsäure (phosphorimidic triamide) hergestellt.

$$1.\quad (R_2N)_3P^+HalHal^- + 2\,NH_3 \xrightarrow{-NH_4Hal} (R_2N)_3P^+NH_2Hal^-$$

$$2.\quad (R_2N)_3P^+NH_2Hal^- \xrightarrow{-H_2O/OH^-} (R_2N)_3P{=}NH$$

Das Triamid der Iminophosphorsäure wird anschließend mit 1,5-Dibrompentan entsprechend den nachfolgenden Reaktionsgleichungen

$$3.\ (R_2N)_3P{=}NH + Br(CH_2)_5Br \rightarrow (R_2N)_3P{=}N\text{-}(CH_2)_5\text{-}Br$$

4. 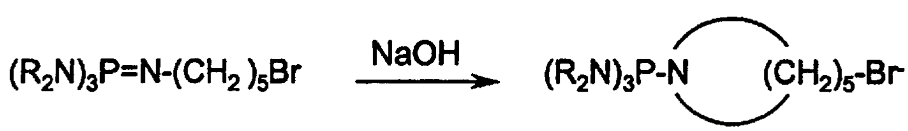


unter Ringschlußreaktion in das Tetrakis(piperidino)phosphoniumbromid überführt.

Diese Art der Synthese ist sehr aufwendig, erfordert mehrere Reaktionsstufen und führt zu einem Reaktionsprodukt, das neben dem Tetrakis(piperidino)phosphonium-bromid stets noch stark basische Verbindungen der Formel $(R_2N)_3P{=}NH$ und $(R_2N)_3P{=}N\text{-}(CH_2)_5Br$ beinhaltet. Diese Verbindungen wirken sich störend auf die katalytische Wirksamkeit, insbesondere auf die Selektivität bestimmter Halex-Reaktionen aus.

Daher besteht ein Bedarf, ein Verfahren zur Herstellung von Tetrakis(piperidino)-phosphoniumsalzen bereitzustellen, das die vorstehenden Nachteile vermeidet, sich auf einfache Art auch technisch realisieren läßt und die gewünschten Produkte in guten Ausbeuten zugänglich macht.

Die Aufgabe wird gelöst durch ein Verfahren zur Herstellung der vorstehend genannten Mischungen enthaltend die Verbindungen $(R)_4P^+X^-$ und $(R)_3P{=}O$. Es ist dadurch gekennzeichnet, daß man ein Phosphorpentahalogenid mit Pyrrolidin oder Piperidin im Molverhältnis 1:6 bis 1:50 in Anwesenheit eines inerten Lösungsmittels zunächst bei 10 bis 80°C umsetzt, anschließend die Reaktion bei 90 bis 180°C fortführt, das erhaltene Reaktionsprodukt bei 0 bis 80°C mit wäßriger Lauge bei einem pH-Wert von 7 bis 15 behandelt und wäßrige und organische Phase voneinander trennt.

Man legt üblicherweise das Phosphorpentahalogenid und das Lösungsmittel vor und setzt anschließend Pyrrolidin oder Piperidin zu, allerdings ist auch die umgekehrte Vorgehensweise möglich. Die Umsetzung läuft dabei zunächst in dem vorstehend genannten Temperaturbereich ab. In der Regel soll die Temperatur in dem angegebenen Temperaturbereich, gegebenenfalls durch Kühlen, gehalten werden. Für eine gute Durchmischung der Reaktionspartner ist zu sorgen. Besonders einfach gestaltet sich dieser Reaktionsschritt, wenn man Pyrrolidin oder Piperidin mit einer solchen Geschwindigkeit zugibt, daß der genannte Temperaturbereich eingehalten wird.

Im Anschluß an diesen Reaktionsschritt wird die Reaktion, wie zuvor angegeben, bei einer höheren Temperatur fortgesetzt, wobei das gewünschte Tetrakis(pyrrolidino) - respektive Tetrakis(piperidino)phosphoniumsalz gebildet wird.

Hierbei begünstigen lange Reaktionszeiten und hohe Reaktionstemperaturen die Bildung der Tetrakis(pyrrolidino)- bzw. Tetrakis(piperidino)phosphoniumsalze, während kürzere Reaktionszeiten und niedrige Reaktionstemperaturen zu Mischungen mit erhöhtem Anteil an Verbindungen $(R)_3P{=}O$ führen.

Nach Beendigung dieser Umsetzung wird das Reaktionsprodukt, wie zuvor bereits erwähnt, bei einer Temperatur von 0 bis 80°C mit einer wäßrigen Lauge behandelt. Man setzt die Lauge in einer solchen Menge ein, daß

während der Behandlung ein pH-Wert von 7 bis 15 eingehalten wird. Durch die Behandlung mit der wäßrigen Lauge werden hydrolysierbare Bestandteile des Reaktionsproduktes hydrolysiert. Als Folge dieser Hydrolyse bilden sich vermutlich die Verbindungen der Formel $(R)_3P=O$ (2), die in unterschiedlichen Mengen anfallen können. Die Behandlung mit wäßriger Lauge bewirkt ferner, daß die im Verlauf der Umsetzung gebildeten Hydrohalogenide des Pyrrolidins respektive Piperidins neutralisiert und Pyrrolidin respektive Piperidin freigesetzt werden. Das freigesetzte Pyrrolidin respektive Piperidin kann zurückgewonnen und in der Reaktion wieder eingesetzt werden.

Man trennt die wäßrige Phase von der organischen Phase, die das gewünschte Reaktionsprodukt, Lösungsmittel, überschüssig eingesetztes und/oder aus den Hydrohalogeniden freigesetztes Pyrrolidin respektive Piperidin enthält. Anschließend engt man die organische Phase beispielsweise durch Vakuumdestillation bis zur Trockene ein. Der dabei anfallende Feststoff enthält die erfindungsgemäßen Mischungen und kann unmittelbar, beispielsweise als Katalysator oder Katalysatorbestandteil, eingesetzt werden.

Im Hinblick auf den durch J. Gen. Chem. USSR (Engl. Transl.) 52, 1982, Seite 1779 bis 1787 beschriebenen Stand der Technik darf es als überraschend angesehen werden, daß sich Tetrakis(piperidino)phosphoniumsalze direkt durch Umsetzung eines Phosphorpentahalogenids mit Piperidin herstellen lassen.

In Anbetracht der bei der erfindungsgemäßen Herstellung angewendeten scharfen Reaktionsbedingungen, insbesondere der hohen Temperaturen ist nicht zu erwarten gewesen, daß sich eine im wesentlichen lediglich zwei Verbindungen enthaltende Mischung herstellen läßt, die einen hohen Anteil von Tetrakis(pyrrolidino) - respektive Tetrakis(piperidino)phosphoniumsalzen besitzt und die sich unmittelbar als Katalysator oder Katalysatorbestandteil einsetzen läßt.

Üblicherweise führen bei hohen Temperaturen ablaufende Umsetzungen zu Reaktionsprodukten, die aus einer Vielzahl verschiedener Reaktionsprodukte bestehen und Verunreinigungen besitzen, die einen Einsatz als Katalysator - ohne eine aufwendige zusätzliche Reinigung vornehmen zu müssen - verhindern. Katalysatorgifte wirken, wie der Fachmann weiß, bereits in sehr geringen Mengen. Die Mischungen enthalten keine störenden Verbindungen $(R)_3P=NR'$ (R'= Wasserstoff, Alkyl, Alkenyl mit 1 bis 6 C-Atomen oder $-(CH_2)_xHal$, wobei x = 2 bis 5 und Hal für Cl oder Br steht). Verbindungen dieses Typs sind als starke Basen bekannt (siehe z.B.: R. Schwesinger et. al., Chem. Ber. 1994, 127, 2435-2454, hier insbesondere Seite 2440).

In einer Vielzahl von Fällen setzt man das Phosphorpentahalogenid mit Pyrrolidin oder Piperidin im Molverhältnis 1:7 bis 1:25, insbesondere 1:8 bis 1:16, bevorzugt 1:8 bis 1:14 um. Man setzt Phosphorpentahalogenid mit Pyrrolidin oder Piperidin, wie eingangs erwähnt, zunächst bei -20 bis 80, insbesondere 20 bis 75, bevorzugt 40 bis 70°C um.

Man setzt als inertes Lösungsmittel einen aliphatischen, cycloaliphatischen oder aromatischen Kohlenwasserstoff oder einen einfach oder mehrfach chlorierten aliphatischen, cycloaliphatischen oder aromatischen Kohlenwasserstoff ein.

Gut geeignet als inertes Lösungsmittel sind z.B. Hexan, Cyclohexan, Methylcyclohexan, Toluol, Ethylbenzol, Mesitylen, o-Xylol, m-Xylol, p-Xylol, technische Gemische isomerer Xylole, Methylenchlorid, Tetrachlorethan, Chlorbenzol, Dichlorbenzol oder Chlortoluol, insbesondere o-Xylol, m-Xylol, p-Xylol, technische Gemische isomerer Xylole, Chlorbenzol. Es lassen sich auch Gemische von Lösungsmitteln verwenden.

Überschüssig eingesetztes Pyrrolidin oder Piperidin eignet sich ebenfalls als Lösungsmittel. In diesem Falle legt man Pyrrolidin oder Piperidin vor und dosiert das in einem inerten Lösungsmittel, nicht jedoch in Pyrrolidin oder Piperidin, suspendierte oder gelöste Phosphorpentahalogenid unter Vermischen zu.

Man führt die Reaktion, wie zuvor bereits genannt, bei 90 bis 180, insbesondere 100 bis 170, bevorzugt 120 bis 150°C fort.

Das Fortführen der Reaktion gestaltet sich besonders einfach, wenn man unter Rückflußbedingungen arbeitet und ein Lösungsmittel wählt, das einen Siedepunkt innerhalb der vorstehend genannten Temperaturbereiche aufweist.

Es ist allerdings auch möglich, die Reaktion unter Druck durchzuführen. Somit lassen sich auch, falls gewünscht, Lösungsmittel mit niedrigeren Siedepunkten einsetzen.

Einer besonderen Variante zufolge setzt man Phosphorpentachlorid oder Phosphorpentabromid, insbesondere Phosphorpentachlorid als Phosphorpentahalogenid ein. Es ist auch möglich, das Phosphorpentahalogenid in einem vorangehenden Reaktionsschritt aus dem entsprechenden Phosphortrihalogenid und dem Halogen herzustellen.

Nach Beendigung der Umsetzung behandelt man das Reaktionsprodukt, wie bereits erwähnt, bei 0 bis 100, insbesondere 10 bis 70, bevorzugt 25 bis 50°C mit wäßriger Lauge bei einem pH-Wert von 7 bis 15, insbesondere 8 bis 14,5, bevorzugt 9 bis 14. Als wäßrige Lauge eignet sich beispielsweise eine 5 bis 50, insbesondere 15 bis 30, bevorzugt 15 bis 25 gew.-%ige wäßrige Alkali- oder Erdalkalihydroxid-Lösung. Besonders einfach ist es, eine entsprechende wäßrige NaOH oder KOH, insbesondere eine NaOH Lösung zu verwenden. In vielen Fällen hat es sich bewährt, eine 20 bis 25 %ige wäßrige NaOH oder KOH, insbesondere NaOH zu verwenden.

Während der Behandlung des Reaktionsproduktes mit der Lauge ist für eine gute Durchmischung zu sorgen. Im Anschluß an die Behandlung des Reaktionsproduktes mit der Lauge wird die wäßrige Phase von der organischen

Phase abgetrennt. In der organischen Phase befinden sich die Mischungen, enthaltend die Verbindungen $(R)_4P^+X^-$ und $(R)_3P{=}O$. Durch Abtrennen der flüchtigen Bestandteile, hierzu zählen das Lösungsmittel und noch vorhandenes Pyrrolidin oder Piperidin, erhält man die Mischungen als Feststoff. Falls gewünscht kann man durch Umkristallisieren den Anteil von Tetrakis(pyrrolidino)-phosphoniumsalzen respektive Tetrakis(piperidino)-phosphoniumsalzen erhöhen. Falls gewünscht, kann $X^-$ = Cl oder Br gegen andere der vorstehend genannten Anionen ausgetauscht werden.

Die Erfindung betrifft ferner die Verwendung der vorstehend näher beschriebenen Mischungen, die die Verbindung der Formel $(R)_4P^+X^-$ (1) und die Verbindung der Formel $(R)_3P{=}O$ (2) enthalten, worin R und X die vorstehend genannte Bedeutung besitzen, als Katalysator oder Cokatalysator für Phasentransferreaktionen, nucleophile Substitutionsreaktionen oder Halogen-Fluor-Austauschreaktionen, insbesondere für Phasentransferreaktionen oder Halogen-Fluor-Austauschreaktionen, bevorzugt für Halogen-Fluor-Austauschreaktionen.

Als Katalysator für Halogen-Fluor-Austauschreaktionen (Halex Reaktionen) eignen sich beispielsweise Stoffgemische enthaltend eine der vorstehend beschriebenen Mischungen, die die Verbindung der Formel $(R)_4P^+X^-$ (1) und die Verbindung der Formel $(R)_3P{=}O$ (2) enthalten, worin R und $X^-$ die vorstehende Bedeutung haben und wenigstens eine Verbindung ausgewählt aus der Gruppe von quartären Ammoniumverbindungen der Formel

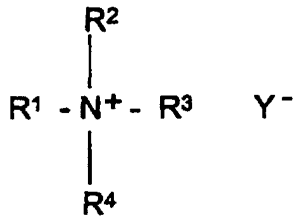

(3),

quartären Ammoniumsalzen oder Phosphoniumsalzen der Formel

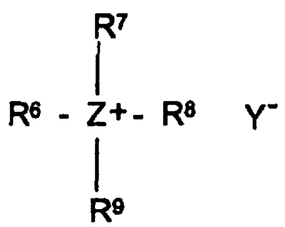

(4),

Polyethem der Formel $R^{10}\text{-}(O\text{-}C_xH_{2x})_s\text{-}OR^{11}$ (5) und Kronenethern, worin in Formel (3) $R^1$, $R^2$ und $R^3$ gleich oder verschieden sind und einen linearen oder verzweigten Rest der Formel $-(C_pH_{2p}O)_rR^5$ bedeuten, worin $R^5$ für Wasserstoff oder einen linearen oder verzweigten Alkylrest mit 1 bis 16 Kohlenstoffatomen steht, p eine ganze Zahl von 1 bis 10 und r eine ganze Zahl von 1 bis 15 bedeuten;
oder einen linearen oder verzweigten Alkylrest mit 1 bis 30 Kohlenstoffatomen;
oder einen unsubstituierten Phenyl- oder Naphthylrest, oder einen substituierten Phenyl- oder Naphthylrest, wobei die Substituenten die Bedeutung Halogen, $C_1$-$C_4$-Alkyl, $C_1$-$C_4$-Alkoxy, Nitro oder Cyano haben;
$R^4$ einen linearen oder verzweigten Rest der Formel $-(C_pH_{2p}O)_rR^5$ bedeutet; und
$Y^-$ ein anorganisches Anion ist;
und in Formel (4)
$R^6$, $R^7$, $R^8$ und $R^9$ gleich oder verschieden sind und einen linearen oder verzweigten Alkylrest mit 1 bis 22 Kohlenstoffatomen bedeuten; oder einen unsubstituierten oder substituierten Arylrest oder einen $C_1$-$C_4$-Alkylarylrest, wobei Aryl die Bedeutung Phenyl oder Naphthyl hat und die besagten Substituenten Halogen,
$C_1$-$C_4$-Alkyl, $C_1$-$C_4$-Alkoxy, Nitro oder Cyano bedeuten; Z die Bedeutung N oder P hat und Y ein anorganisches Anion ist;
und in Formel (5)
$R^{10}$ und $R^{11}$ gleich oder verschieden sind und einen linearen oder verzweigten Alkylrest mit 1 bis 16 Kohlenstoffatomen bedeuten;
x eine ganze Zahl von 2 bis 6 und

s eine ganze Zahl von 1 bis 60 ist;
oder einer der Reste $R^{10}$ und $R^{11}$ Wasserstoff und der andere der Reste einen linearen oder verzweigten Alkylrest mit 1 bis 16 Kohlenstoffatomen bedeutet,
x eine ganze Zahl von 2 bis 6 und s eine ganze Zahl von 2 bis 50 ist, oder die Reste $R^{10}$ und $R^{11}$ Wasserstoff bedeuten, x eine ganze Zahl von 2 bis 6 und s eine ganze Zahl von 3 bis 5 ist.

Als Katalysator für Halogen-Fluor-Austauschreaktionen eignen sich Stoffgemische enthaltend eine der vorstehend beschriebenen Mischungen, die die Verbindung $(R)_4P^+X^-$ und die Verbindung $(R)_3P{=}O$ enthalten, und wenigstens eine Verbindung ausgewählt aus der Gruppe von quartären Ammoniumverbindungen der Formel (3), quartären Ammoniumsalzen und Phosphoniumsalzen der Formel (4), Polyethern der Formel (5) und Kronenethern, worin in Formel (3) $R^1$, $R^2$ und $R^3$ gleich oder verschieden sind und einen linearen oder verzweigten Rest der Formel $-(C_pH_{2p}O)_rR^5$ bedeuten, worin $R^5$ für Wasserstoff oder einen linearen oder verzweigten Alkylrest mit 1 bis 8 Kohlenstoffatomen steht, p eine ganze Zahl von 1 bis 5 und r eine ganze Zahl von 2 bis 10 bedeuten; oder einen linearen oder verzweigten Alkylrest mit 1 bis 18 Kohlenstoffatomen; oder einen unsubstituierten Phenyl- oder Naphthylrest; $R^4$ einen linearen oder verzweigten Rest der Formel $-(C_pH_{2p}O)_rR^5$ bedeutet, worin $R^5$ für Wasserstoff oder einen linearen oder verzweigten Alkylrest mit 1 bis 8 Kohlenstoffatomen steht, p eine ganze Zahl von 1 bis 5 und r eine ganze Zahl von 2 bis 10 bedeuten.
Als Katalysator für Halogen-Fluor-Austauschreaktionen sind besonders von Interesse Stoffgemische, die eine der vorstehend beschriebenen Mischungen und wenigstens eine Verbindung aus der Gruppe der quartären Ammoniumverbindungen der Formel (3) enthalten.

Die Stoffgemische enthalten üblicherweise 5 bis 95 Gew.-%, insbesondere 20 bis 80 Gew.-%, bevorzugt 25 bis 75 Gew.-% einer Mischung enthaltend die Verbindung $(R)_4P^+X^-$ und $(R)_3P{=}O$. Die verbleibenden 95 bis 5 Gew.-%, insbesondere 80 bis 20 Gew.-%, bevorzugt 75 bis 25 Gew.-% der Stoffgemische entfallen auf den Rest, nämlich auf wenigstens eine Verbindung ausgewählt aus der Gruppe der quartären Ammoniumverbindungen der Formel (3), der quartären Ammonium- oder Phosphoniumsalze der Formel (4), Polyethem der Formel (5) und Kronenethem, insbesondere auf wenigstens eine Verbindung aus der Gruppe der quartären Ammoniumverbindungen der Formel (3).

Die nachfolgenden Beispiele beschreiben die Erfindung näher, ohne sie zu beschränken.

Experimenteller Teil

Beispiel 1:

Herstellung einer Mischung A enthaltend Tetrakis(pyrrolidino)phosphoniumchlorid und Tris(pyrrolidino)phosphinoxid

104,12 g (0,5 mol) Phosphorpentachlorid werden bei 50°C in 700 g trockenem Xylol gelöst. Dann werden 426,7 g (6 mol) Pyrrolidin so zugetropft, daß die Innentemperatur 75°C nicht überschreitet. Nach der Zugabe wird für 15 Stunden zum Rückfluß erhitzt. Dann wird auf 40°C gekühlt und mit 800 g (4 mol) 20%iger wässriger Natronlauge hydrolysiert. Nach Abtrennen der wäßrigen Phase wird die organische Phase am Rotationsverdampfer bis zur Trockene eingeengt. Aus dem Destillat wird Pyrrolidin redestilliert und erneut eingesetzt. Man erhält 172,8 g eines leicht bräunlichen Feststoffes, der zu 94% aus Tetrakis(pyrrolidino)phosphoniumchlorid, zu 5% aus Tris(pyrrolidino)phosphinoxid und zu 1% aus nicht identifizierten Bestandteilen besteht (Mischung A). Das so erhaltene Produkt wird direkt als Katalysator und Cokatalysator für Halex-Reaktionen verwendet (siehe auch Beispiele 3 und 4).
Elementaranalyse: C=55,4%, H=9,3%, Cl=9,8%, N=16,1%, O=0,3%, P=9,0%
Zur Isolierung der reinen Komponenten wird diese Rohmischung in Tetrahydrofuran (THF) ausgekocht, nach Filtration gewinnt man 161 g (93% d.Th.) Tetrakis(pyrrolidino)phosphoniumchlorid als farbloses Pulver. Aus der THF-Phase wird Tris(pyrrolidino)phosphinoxid durch Kugelrohrdestillation isoliert. Durch Mischen der reinen Komponenten lassen sich Mischungen beliebiger Zusammensetzung herstellen.
Tetrakis(pyrrolidino)phosphoniumchlorid rein: $^1$H-NMR: 8 = 3.19 (m, 16H, $NC\underline{H_2}$), 1.87 (m, 16H, $NCH_2C\underline{H_2}$); $^{13}$C-NMR: $\delta$ = 46.43 (2 C, $J_{P,C}$ [Hz] = 4.8, $N\underline{C}H_2$), 25.64 (2 C, $J_{P,C}$ [Hz] = 8.3, $NCH_2\underline{C}H_2$), $^{31}$P-NMR: $\delta$ = 26.0 (s, 1P).

Beispiel 2:

Herstellung einer Mischung B enthaltend Tetrakis(piperidino)phosphoniumchlorid und Tris(piperidino)phosphinoxid

Tetrakis(piperidino)phosphoniumchlorid wird analog Beispiel 1 aus 104,12 g (0,5 mol) Phosphorpentachlorid in 730 g Chlorbenzol als Lösungsmittel und 510,9 g (6 mol) Piperidin hergestellt.
Man erhält 197,8 g eines leicht bräunlichen Pulvers, das zu 92% aus
Tetrakis(piperidino)phosphoniumchlorid, zu 7% aus Tris(piperidino)phosphinoxid und zu 1% aus nicht identifizierten Bestandteilen besteht (Mischung B). Das so erhaltene Produkt wird direkt als Katalysator und Cokatalysator für Halex-Reaktionen verwendet (siehe auch Beispiele 3 und 4).
Elementaranalyse: C=67,54%, H=11,63%, Cl=9,84%, N=16,16%, O=0,23%, P=9,04%
Die Isolierung der reinen Komponenten erfolgt analog Beispiel 1.
Tetrakis(piperidino)phosphoniumchlorid rein: $^1$H-NMR: δ = 3.14 (m, 16H, NC<u>H</u>$_2$), 1.71 (m, 8H, $NCH_2CH_2C\underline{H}_2$), 1.64 (m, 16H, $NCH_2C\underline{H}_2$); $^{13}$C-NMR: δ = 47.03 (2 C, $N\underline{C}H_2$), 25.73 (2 C, $NCH_2\underline{C}H_2$), 23.61 (2 C, $NCH_2CH_2\underline{C}H_2$), $^{31}$P-NMR: δ = 38.8 (s, 1P).

Vergleichsbeispiel A:

Herstellung von Tetrakis(diethylamino)phosphoniumbromid (Vergleichssubstanz)

Zu 52,1 g (0,25 mol) $PCl_5$ in 220 ml Chlorbenzol tropft man in 1 Stunde 109,7 g (1,5 mol) Diethylamin so zu, daß die Innentemperatur 10°C nicht übersteigt. Nach der Zugabe wird 1 Stunde bei 30°C nachgerührt, dann wird bei T=15°C 30 g Ammoniak eingeleitet.
Nach 1 Stunde werden 340 g 20%ige wässrige Natronlauge zugegeben und die wässrige Phase abgetrennt. Von der organischen Phase wird das überschüssige Diethylamin abdestilliert. Dann wird mit 170 g 50%iger Natronlauge und 60 g (0,55 mol) Ethylbromid versetzt und für 4 Stunden auf 50°C erwärmt. Nach Phasentrennung wird die organische Phase mit 60%iger Bromwasserstoffsäure auf pH=6 bis 7 angesäuert. Nach Abdestillieren aller flüchtigen Bestandteile erhält man 83,9 g Tetrakis(diethylamino)phosphoniumbromid als leicht bräunliches Öl. Als Verunreinigung sind 1,5 Gew-% des Imino-tris(dialkylamino)phosphorans $(Et_2N)_3P{=}NEt$ enthalten. Das so erhaltene Produkt (Vergleichssubstanz) wird direkt als Katalysator und Cokatalysator für Halex-Reaktionen verwendet (siehe auch Beispiele 3 und 4).

Vergleichsbeispiel B:

Versuch, $PCl_5$ und Diethylamin zu Tetrakis(diethylamino)phosphoniumchlorid umzusetzen

Zu 20.8 g (0,1 mol) $PCl_5$ in 180 ml Xylol tropft man bei T<15°C 73,1 g (1,0 mol) Diethylamin. Nach der Zugabe wird für 2 Stunden zum Rückfluß erhitzt. Nach Abkühlen auf 22°C wird mit 160 g 25%iger wässriger Natronlauge hydrolysiert. Die Hydrolyse ist stark exotherm (Temperaturanstieg auf 48°C). Die wässrige Phase wird abgetrennt und die verbleibende organische Phase am Rotationsverdampfer eingeengt. Nach Kugelrohrdestillation erhält man 24,9 g Tris(diethylamino)-phosphinoxid als gelbes Öl. Eine Bildung von Tetrakis(diethylamino)-phosphoniumchlorid wird nicht beobachtet.

Vergleichsbeispiel C:

Herstellung von Tetrakis(piperidino)phosphoniumbromid

41,65 g (0,2 mol) Phosphorpentachlorid werden in 220 g Chlorbenzol vorgelegt und bei 5°C mit 102,18 g (1,2 mol) Piperidin versetzt. Nach 30 min Reaktionszeit wird bei 10°C 40 g Ammoniak eingeleitet. Nach 30 Minuten wird bei 40°C mit 130 g Natronlauge (50%-ige wäßrige Lösung) hydrolysiert. Die wäßrige Phase wird abgetrennt und die organische Phase bei 120°C/10 mbar von flüchtigen Bestandteilen befreit. Der Rückstand wird bei 0,1 mbar über eine Kurzwegkolonne destilliert. Es werden 37 g (0,124 mol) Tris(piperidino)phosphorimin $(Pip)_3P{=}NH$ (Mw.: 298,41) destilliert.
14,92 g (0,05 mol) des so erhaltenen Tris(piperidino)phosphorimins werden in 100 ml 40%iger wäßriger Natronlauge vorgelegt. Dann tropfen in 20 min 12,65 g (0,055 mol) 1,5-Dibrompentan zu. Nach zweistündigem Nachrühren bei 20°C wird für eine Stunde auf 100°C erwärmt. Nach dem Abkühlen wird die Mischung mit 3 x 20 ml Methylenchlorid extrahiert. Die organischen Extrakte werden über Natriumsulfat getrocknet. Nach Verdampfen des Lösungsmittels am Rotationsverdampfer erhält man 21,4 g (0,48 mol) Tetrakis(piperidino)phosphoniumbromid.

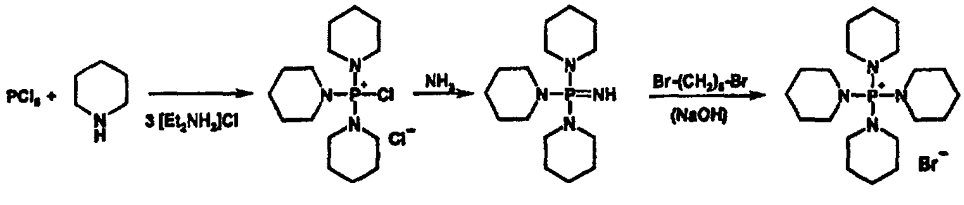

Beispiel 3: Synthese von 4-Fluorbenzaldehyd

Zu 421,7 g (3 mol) 4-Chlorbenzaldehyd (4-CBAL) werden nacheinander bei 60°C eine Lösung von 12,0 g (0,03 mol) Tetrakis(diethylamino)phosphoniumbromid in 12 g N-Methylpyrrolidon (NMP), 174,3 g (3 mol) Kaliumfluorid, 4,1 g (0,03 mol) Nitrotoluol und 18 ml Xylol gegeben. Durch Abdestillieren des Xylols bei vermindertem Druck wird die Reaktionsmischung azeotrop getrocknet. Nach 24 Stunden bei 190°C wird die Bildung von 4-Fluorbenzaldehyd (4-FBAL), durch Dehalogenierung entstandenem Benzaldehyd und der Umsatz der Reaktion gaschromatographisch bestimmt.

In den erfindungsgemäßen Beispielen wird anstelle von Tetrakis-(diethylamino)-phosphoniumbromid eine molar entsprechende Menge (jeweils 0,03 mol) des jeweils angegebenen Aminophosphoniumsalzes in Form von Mischung A und B (hergestellt gemäß Beispiel 1 und 2) eingesetzt. Sonst wird analog verfahren.
Die Angaben in Tabelle 1 entsprechen GC-Flächenprozent. Die in Tabelle 1 als Rest angegebene Differenz zu 100% ist ein Maß für Nebenreaktionen und Zersetzung.

Tabelle 1:

| Ausbeuten von 4-Fluorbenzaldehyd (4-FBAL) aus der Halex-Reaktion von 4-Chlorbenzaldehyd (4-CBAL) in GC-Flächen-% | | | | |
|---|---|---|---|---|
| Aminophosphoniumsalz | 4-FBAL | 4-CBAL | Benzaldehyd | Rest |
| (($Et_2N)_4P$]Br* | 47,8 | 34,5 | 0,23 | 17,5 |
| [(Pyrrolidino)$_4$P]Cl** | 56,7 | 29,6 | 0,03 | 14,1 |
| [(Piperidino)$_4$P]Cl*** | 54,2 | 32,5 | 0,02 | 13,3 |
| [(Piperidino)$_4$P]Br**** | 42,9 | 38,9 | | 18,2 |

* Vergleichsbeispiel unter Verwendung der aus Vergleichsbeispiel A erhaltenen Vergleichssubstanz
** Erfindungsgemäßes Beispiel unter Verwendung der aus Beispiel 1 erhaltenen Mischung A
*** Erfindungsgemäßes Beispiel unter Verwendung der aus Beispiel 2 erhaltenen Mischung B
**** Vergleichsbeispiel unter Verwendung der aus Vergleichsbeispiel C erhaltenen Vergleichssubstanz

Beispiel 4:

Synthese von 2-Chlor-6-fluorbenzaldehyd und 2,6-Difluorbenzaldehyd

Zu 175,0 g (1 mol) 2,6-Dichlorbenzaldehyd (DCBAL) werden nacheinander bei 60°C 8,8 g (0,02 mol) Tetrakis-(diethylamino)phosphoniumbromid, 35,2 g (0,06 mol) Methyl-tris-(methyltetraethoxy)-ammonlumchlorid [{$CH_3$-{$O-C_2H_4)_4\}_3NCH_3$]Cl, 72,6 g (1,25 mol) Kaliumfluorid und 10 ml Xylol zugegeben. Durch Abdestillieren des Xylols bei vermindertem Druck wird die Reaktionsmischung azeotrop getrocknet. Nach 20 Stunden bei 165°C wird die Bildung von 2-Chlor-6-fluorbenzaldehyd (CFBAL), 2,6-Difluorbenzaldehyd (DFBAL), durch Dehalogenierung entstandenem o-Chlorbenzaldehyd (o-CBAL) und der Umsatz der Reaktion gaschromatographisch bestimmt.

In den erfindungsgemäßen Beispielen wird anstelle von Tetrakis-(diethylamino)phosphoniumbromid eine molar entsprechende Menge des jeweils angegebenen Aminophosphoniumsalzes (jeweils 0,02 mol) in Form von Mischung A und B (hergestellt gemäß Beispiel 1 und 2) eingesetzt. Sonst wird analog verfahren. Die Angaben in Tabelle 2 entsprechen GC-Flächenprozent. Die in Tabelle 2 als Rest angegebene Differenz zu 100% ist ein Maß für Nebenreaktionen und Zersetzung.

Tabelle 2:

| Ausbeuten von 2,6-Difluorbenzaldehyd (DFBAL) und 2-Chlor-6-fluorbenzaldehyd (CFBAL) aus der Halex-Reaktion von 2,6-Dichlorbenzaldehyd (DCBAL) in GC-Flächen-% | | | | | |
|---|---|---|---|---|---|
| Aminophosphoniumsalz | DFBAL | CFBAL | DCBAL | o-CBAL | Rest |
| [($Et_2N)_4P$]Br* | 32,1 | 37,0 | 10,1 | 0,81 | 20,0 |
| [(Pyrrolidino)$_4$P]Cl** | 33,6 | 42,0 | 5,4 | 0,24 | 18,7 |

* Vergleichsbeispiel unter Verwendung der aus Vergleichsbeispiel A erhaltenen Vergleichssubstanz
** Erfindungsgemäßes Beispiel unter Verwendung der aus Beispiel 1 erhaltenen Mischung A

Tabelle 2: (fortgesetzt)

| Ausbeuten von 2,6-Difluorbenzaldehyd (DFBAL) und 2-Chlor-6-fluorbenzaldehyd (CFBAL) aus der Halex-Reaktion von 2,6-Dichlorbenzaldehyd (DCBAL) in GC-Flächen-% | | | | | |
|---|---|---|---|---|---|
| Aminophosphoniumsalz | DFBAL | CFBAL | DCBAL | o-CBAL | Rest |
| [(Piperidino)4P]Cl*** | 33,3 | 44,4 | 4,9 | 0,19 | 17,2 |
| [(Piperidino)$_4$P]Br**** | 19,4 | 41,4 | 19,8 | 0,52 | 18,9 |

*** Erfindungsgemäßes Beispiel unter Verwendung der aus Beispiel 2 erhaltenen Mischung B

**** Vergleichsbeispiel unter Verwendung der aus Vergleichsbeispiel C erhaltenen Vergleichssubstanz

**Patentansprüche**

1. Mischungen enthaltend 70 bis 99,5 Gew.-% einer Verbindung der Formel $(R)_4P^+X^-$ (1) und 30 bis 0,5 Gew.-% einer Verbindung der Formel $(R)_3P{=}O$ (2), wobei R jeweils für einen Rest

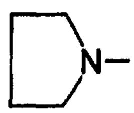

oder

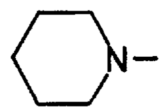

und $X^-$ für ein anorganisches oder organisches Anion oder das Äquivalent eines mehrwertigen anorganischen oder organischen Anions steht.

2. Mischungen enthaltend 75 bis 99 Gew.-% der Verbindung $(R)_4P^+X^-$ und 25 bis 1 Gew.-% der Verbindung $(R)_3P{=}O$.

3. Mischungen enthaltend 80 bis 98 Gew.-% der Verbindung $(R)_4P^+X^-$ und 20 bis 2 Gew.-% der Verbindung $(R)_3P{=}O$.

4. Mischungen nach einem oder mehreren der Ansprüche 1 bis 3, **dadurch gekennzeichnet, daß** $X^-$ für $F^-$, $Cl^-$, $Br^-$, $J^-$, $ClO_4^-$, $BF_4^-$, $PF_6^-$, $NO_3^-$, $HSO_4^-$, ½ $SO_4^{2-}$, $H_2PO_4^-$, $^1/_3$ $PO_4^{3-}$, R"COO$^-$, wobei R" einen Alkylrest mit 1 bis 9 C-Atomen, einen Phenylrest, Benzylrest oder Naphtylrest bedeutet, R'"-$SO_3^-$, wobei R'" einen Alkylrest mit 1 bis 18 C-Atomen, einen Phenylrest, Tolylrest oder Naphthylrest bedeutet, $HCO_3$, ½ $CO_3^{2-}$ oder für ½ $C_6H_4(COO^-)_2$ steht.

5. Verfahren zur Herstellung von Mischungen nach einem der Ansprüche 1 bis 4, **dadurch gekennzeichnet, daß** man ein Phosphorpentahalogenid mit Pyrrolidin oder Piperidin im Molverhältnis 1:6 bis 1:50 in Anwesenheit eines inerten Lösungsmittels zunächst bei -20 bis 80°C umsetzt, anschließend die Reaktion bei 90 bis 180°C fortführt, das erhaltene Reaktionsprodukt bei 0 bis 80°C mit wäßriger Lauge bei einem pH-Wert von 7 bis 15 behandelt und wäßrige und organische Phase voneinander trennt.

6. Verfahren nach Anspruch 5, **dadurch gekennzeichnet, daß** man das Phosphorpentahalogenid mit Pyrrolidin oder Piperidin im Molverhältnis 1:8 bis 1:25 umsetzt.

7. Verfahren nach Anspruch 5 oder 6, **dadurch gekennzeichnet, daß** man als inertes Lösungsmittel einen aliphatischen, cycloaliphatischen oder aromatischen Kohlenwasserstoff oder einen einfach oder mehrfach chlorierten aliphatischen, cycloaliphatischen oder aromatischen Kohlenwasserstoff, einsetzt.

8. Verfahren nach einem oder mehreren der Ansprüche 5 bis 7, **dadurch gekennzeichnet, daß** man das Phosphorpentahalogenid mit Pyrrolidin oder Piperidin zunächst bei 20 bis 75°C umsetzt.

9. Verfahren nach einem oder mehreren der Ansprüche 5 bis 8, **dadurch gekennzeichnet, daß** man die Reaktion bei 100 bis 170°C fortführt.

10. Verfahren nach einem oder mehreren der Ansprüche 5 bis 9, **dadurch gekennzeichnet, daß** man Phosphorpentachlorid als Phosphorpentahalogenid einsetzt.

**11.** Verwendung der Mischungen nach einem der Ansprüche 1 bis 4 als Katalysator und Cokatalysator für Phasentransferreaktionen, nucleophile Substitutionsreaktionen oder Halogen-Fluor-Austauschreaktionen.

## Claims

**1.** A mixture comprising from 70 to 99.5% by weight of a compound of the formula $(R)^4P^+X^-$ (1) and from 30 to 0.5% by weight of a compound of the formula $(R)_3P=O$ (2), where R is in each case a radical

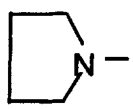

or

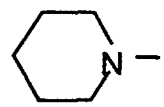

and $X^-$ is an inorganic or organic anion or the equivalent of a multiply charged inorganic or organic anion.

**2.** A mixture comprising from 75 to 99% by weight of the compound $(R)_4P^+X^-$ and from 25 to 1% by weight of the compound $(R)_3P=O$.

**3.** A mixture comprising from 80 to 98% by weight of the compound $(R)_4P^+X^-$ and from 20 to 2% by weight of the compound $(R)_3P=O$.

**4.** A mixture as claimed in one or more of claims 1 to 3, wherein $X^-$ is $F^-$, $Cl^-$, $Br^-$, $I^-$, $ClO_4^-$, $BF_4^-$, $PF_6^-$, $NO_3^-$, $HSO_4^-$, ½ $SO_4^{2-}$, $H_2PO_4^-$, $1/_3$ $PO_4^{3-}$, R''COO⁻, where R'' is an alkyl radical having 1 to 9 carbon atoms, a phenyl radical, benzyl radical or naphthyl radical, $R'''-SO_3^-$, where R''' is an alkyl radical having 1 to 18 carbon atoms, a phenyl radical, a tolyl radical or a naphthyl radical, $HCO_3^-$, ½ $CO_3^{2-}$ or % $C_6H_4(COO^-)_2$.

**5.** A process for preparing mixtures as claimed in any of claims 1 to 4, which comprises reacting a phosphorus pentahalide with pyrrolidine or piperidine in the molar ratio 1:6 to 1:50 in the presence of an inert solvent initially at -20 to 80°C, subsequently continuing the reaction at 90 to 180°C, treating the resulting reaction product at 0 to 80°C with aqueous alkali at a pH of 7 to 15, and separating aqueous and organic phase from one another.

**6.** The process as claimed in claim 5, wherein the phosphorus pentahalide is reacted with pyrrolidine or piperidine in the molar ratio 1:8 to 1:25.

**7.** The process as claimed in claim 5 or 6, wherein an aliphatic, cycloaliphatic or aromatic hydrocarbon or a mono- or polychlorinated aliphatic, cycloaliphatic or aromatic hydrocarbon is employed as inert solvent.

**8.** The process as claimed in one or more of claims 5 to 7, wherein the phosphorus pentahalide is reacted with pyrrolidine or piperidine initially at 20 to 75°C.

**9.** The process as claimed in one or more of claims 5 to 8, wherein the reaction is continued at 100 to 170°C.

**10.** The process as claimed in one or more of claims 5 to 9, wherein phosphorus pentachloride is employed as phosphorus pentahalide.

**11.** The use of the mixtures as claimed in any of claims 1 to 4 as catalyst and cocatalyst for phase transfer reactions, nucleophilic substitution reactions or halogen-fluorine exchange reactions.

## Revendications

**1.** Mélanges contenant de 70 à 99,5 % en poids d'un composé de formule $(R)_4P^+X^-$ (1) et de 30 à 0,5 % en poids d'un composé de formule $(R)_3P=O$ (2), où R représente toujours un reste de formule

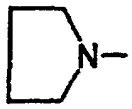 ou 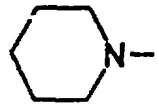

et $X^-$ représente un anion inorganique ou organique ou l'équivalent d'un anion inorganique ou organique multivalent.

2. Mélanges contenant de 75 à 99 % en poids du composé $(R)^4P^+X^-$ et de 25 à 1 % en poids du composé $(R)_3P=O$.

3. Mélanges contenant de 80 à 98 % en poids du composé $(R)_4P^+X^-$ et de 20 à 2 % en poids du composé $(R)_3P=O$.

4. Mélanges selon une ou plusieurs revendications 1 à 3, **caractérisés en ce que** $X^-$ représente des groupes $F^-$, $Cl^-$, $Br^-$, $J^-$, $ClO_4^-$, $BF_4^-$, $PF_6^-$, $NO_3^-$, $HSO_4^-$, 1/2 $SO_4^{2-}$, $H_2PO_4^-$, 1/3 $PO_4^{3-}$, R''-$COO^-$, où R'' représente un reste alkyle présentant 1 à 9 atomes de carbone, un reste phényle, un reste benzyle ou un reste naphtyle, R'''-$SO_3^-$, R''' représente un reste alkyle présentant 1 à 18 atomes de carbone, un reste phényle, un reste tolyle ou un reste naphtyle, $HCO_3^-$, 1/2 $CO_3^{2-}$ ou 1/2 $C_6H_4(COO^-)_2$.

5. Procédé pour la préparation de mélanges selon l'une des revendications 1 à 4, **caractérisé en ce qu'**on fait réagir un penta-halogènure de phosphore avec la pyrrolidine ou la pipéridine dans un rapport molaire de 1:6 à 1:50 en présence d'un solvant inerte d'abord à une température de -20 à 80°C, ensuite on poursuit la réaction à une température de 90 à 180°C, on traite le produit réactionnel obtenu à une température de 0 à 80°C par une lessive aqueuse à une valeur de pH de 7 à 15 et on sépare les phases aqueuse et organique.

6. Procédé selon la revendication 5, **caractérisé en ce qu'**on fait réagir un pentahalogénure de phosphore avec la pyrrolidine ou la pipéridine dans un rapport molaire de 1:8 à 1:25.

7. Procédé selon la revendication 5 ou 6, **caractérisé en ce qu'**on utilise comme solvant inerte un hydrocarbure aliphatique, cycloaliphatique ou aromatique ou un hydrocarbure aliphatique, cycloaliphatique ou aromatique chloré une ou plusieurs fois.

8. Procédé selon une ou plusieurs des revendications 5 à 7, **caractérisé en ce qu'**on fait réagir le pentahalogénure de phosphore avec la pyrrolidine ou la pipéridine, d'abord à une température de 20 à 75°C.

9. Procédé selon une ou plusieurs des revendications 5 à 8, **caractérisé en ce qu'**on met en oeuvre la réaction à une température de 100 à 170°C.

10. Procédé selon une ou plusieurs des revendications 5 à 9, **caractérisé en ce qu'**on utilise le pentachlorure de phosphore en tant que pentahalogénure de phosphore.

11. Utilisation des mélanges selon l'une des revendications 1 à 4 en tant que catalyseur et cocatalyseur dans des réactions de transfert de phase, réactions de substitution nucléophile ou réactions d'échange halogène-fluor.